# EUROPÄISCHE PATENTSCHRIFT

(11) **EP 2 445 660 B1**
(45) Veröffentlichungstag und Bekanntmachung des Hinweises auf die Patenterteilung: **19.02.2014**
(21) Anmeldenummer: 11731292.6
(22) Anmeldetag: 15.06.2011
(51) Int. Cl.: B21C 37/02, B21K 23/00, A61K 6/04, B21H 8/00

(54) **BARREN AUS EDELMETALL UND VERFAHREN ZUR HERSTELLUNG**
BAR MADE OF NOBLE METAL, AND PRODUCTION METHOD
LINGOTS DE MÉTAL PRÉCIEUX ET PROCÉDÉ DE FABRICATION

(30) Priorität: 20.11.2010 DE 102010044199; 15.06.2010 DE 102010030128
(43) Veröffentlichungstag der Anmeldung: 02.05.2012
(60) Teilanmeldung: 13174247.0
(73) Patentinhaber: ESG Edelmetall-Service GmbH&Co. KG, 76287 Rheinstetten (DE)
(72) Erfinder: LOCHMANN, Dominik, 76287 Rheinstetten (DE)
(74) Vertreter: Sommer, Peter
(86) Internationale Anmeldenummer: PCT/EP2011/059879
(87) Internationale Veröffentlichungsnummer: WO 2011/107616

(56) Entgegenhaltungen:
- WO-A1-00/16932
- DE-A1- 2 107 213
- DE-A1- 3 040 052
- DE-T2- 69 415 531
- FR-A- 348 205
- FR-A- 1 152 976
- GB-A- 939 303
- JP-A- 1 133 395
- JP-A- 3 019 393

## Beschreibung

Die Erfindung betrifft einen Barren aus Edelmetall, beispielsweise Gold, Silber, Platin, Palladium oder Legierungen davon und ein Verfahren zur Herstellung eines solchen Barren (siehe z.B. WO 00/16932).

### Stand der Technik

Dosierbare Zahngoldmaterialien sind im Handel als Plättchen erhältlich, wobei die Plättchen durch Herstellung von identischen Abschnitten aus einem gewalzten Goldblech erhalten wurden. Das Goldblech wird durch Auswalzen eines legierten Goldbarrens bereitgestellt, so beschrieben in der DE 10 2004 060 730 A1.

Bei der Herstellung von Goldbarren in kleiner Stückelung, also 1 Gramm, 5 Gramm, 10 Gramm ist es bekannt, auf jedem einzelnen Barren das Gewicht, der Hersteller, die Reinheit des Metalls und das Metall einzuprägen. Barren aus Edelmetall in kleiner Stückelung sind bei Anlegern zwar sehr beliebt, einzeln in der Herstellung aber im Verhältnis zum Metallpreis teuer.

### Darstellung der Erfindung

Der Grundgedanke der Erfindung besteht darin, anstelle vieler einzelner Kleinbarren mit jeweils einer vorgegebenen Masse mk, mehrere solcher Kleinbarren aus einer Tafel oder aus einem Barren mit einer Masse mB auf einmal herzustellen und diese Tafel oder diesen Barren dann zur Ausbildung der Kleinbarren ganz oder teilweise stofflich von einander zu trennen, wobei die Kleinbarren in einer n x m-Anordnung mit n, m ≥ 2 angeordnet sind. Insgesamt ergibt sich also wegen n x m x mk die Masse mB des Barrens.

Dadurch, dass zunächst ein Barren in Form einer Tafel mit einer Sollmasse mB hergestellt wird, der dann, beispielsweise durch Prägen, in viele Kleinbarren aufgeteilt wird, die im Bedarfsfall ohne Verwendung von Werkzeug von einander abzutrennen bzw. von einem Träger zu lösen sind, lassen sich sowohl die Herstellungskosten verringern als auch die Handhabung der Kleinbarren verbessern.

Die Erfindung betrifft daher einen Barren aus Edelmetall oder einer Edelmetall aufweisenden Legierung mit einer Masse mB, wobei der Barren in n x m Kleinbarren mit jeweils einer vorgegebenen Masse mk unterteilt ist, wobei n, m jeweils eine natürliche Zahl ≥ 2 ist. Zwischen den unmittelbar benachbarten Kleinbarren besteht eine stoffliche Verbindung, sodass die Kleinbarren mit ihren unmittelbaren Nachbarn fest verbunden sind. Die stoffliche Verbindung kann beispielsweise in Form einer Brücke oder als Verbindungssteg ausgebildet sein.

Vorteilhafterweise kann die stoffliche Verbindung eine Sollbruchstelle aufweisen. Dabei ist die Biegefestigkeit der Verbindung vorzugsweise so groß, dass ein Verbiegen unter Einwirkung der Schwerkraft vermieden wird und höchstens so groß, dass ein Zerstören der stofflichen Verbindung von Hand durch Biegen oder Brechen möglich ist.

Zusätzlich zu der stofflichen Verbindung oder alternativ dazu kann auf einer Unterseite des Barren ein Trägermaterial angebracht sein, so dass bei der Herstellung der Kleinbarren aus einem Barren oder aus einer Tafel anstelle einer stofflichen Verbindung mit oder ohne Sollbruchstelle eine vollständige Durchtrennung vorgenommen werden kann.

Die stoffliche Verbindung kann vorteilhafterweise Teil einer in den Barren eingebrachten Vertiefung sein. Dabei kann eine Vertiefung auf einer Oberseite und eine Vertiefung gegenüberliegend auf einer Unterseite ausgebildet sein und die stoffliche Verbindung kann zu der Oberseite und zu der Unterseite des Barrens beabstandet sein. Die Vertiefung kann als Rille ausgebildet sein.

Ein weiterer Gegenstand der Erfindung ist ein Barren aus Edelmetall oder einer Edelmetall aufweisenden Legierung, mit einer Masse mB, der in n x m Kleinbarren mit jeweils einer vorgegebenen Masse mk unterteilt ist, wobei n, m jeweils eine natürliche Zahl ≥ 2 ist und wobei auf einer Unterseite des Barren ein Träger angebracht ist und wobei die Kleinbarren umfangseitig vollständig zu den benachbarten Kleinbarren frei sind und ausschließlich an dem Träger befestigt sind.

Vorteilhafterweise können die Kleinbarren mittels einer den Barren bis zum Träger durchdringenden Vertiefung von einander abgetrennt sein.

Besonders vorteilhaft ist es, wenn die Vertiefung eingeprägt ist, da hierdurch eine wirtschaftliche Herstellung möglich ist.

Ein weiterer Gegenstand der Erfindung ist ein Verfahren zur Herstellung eines Barren mit einer Masse mB aus Edelmetall oder einer Edelmetall aufweisenden Legierung, bei dem der Barren in einem Herstellungsschritt in n x m Kleinbarren mit jeweils einer vorgegebenen Masse mk unterteilt wird, wobei n, m jeweils eine natürliche Zahl ≥ 2 ist, wobei zwischen den unmittelbar benachbarten Kleinbarren eine stoffliche Verbindung bestehen bleibt.

Die stoffliche Verbindung kann als Teil einer Vertiefung ausgebildet sein.

Noch ein weiterer Gegenstand der Erfindung ist ein Verfahren zur Herstellung eines Barren mit einer Masse mB aus Edelmetall oder einer Edelmetall aufweisenden Legierung, bei dem der Barren in einem Herstellungsschritt mit einem Träger verbunden wird und in einem weiteren Herstellungsschritt in n x m Kleinbarren mit jeweils einer vorgegebenen Masse mk unterteilt wird, wobei n, m jeweils eine natürliche Zahl ≥ 2 ist, wobei die Kleinbarren umfangseitig vollständig zu den benachbarten Kleinbarren frei werden und ausschließlich an dem Trägermaterial befestigt sind.

Vorteilhafterweise kann der Barren zur Aufteilung in Kleinbarren geprägt sein.

Noch ein weiterer Gegenstand der Erfindung ist ein Verfahren zur Herstellung eines Barren mit einer Masse mB aus Edelmetall oder einer Edelmetall aufweisenden Legierung, bei dem ein Edelmetall-Endlosband in einer entsprechenden Dicke gewalzt wird und einer Umformeinrichtung schrittweise zugeführt und nach einer Umformung das Band weitergeschoben wird. Bei der Umformung wird das Endlosband in eine Reihe aus n x 1 Kleinbarren mit jeweils einer vorgegebenen Masse mk unterteilt, wobei n eine natürliche Zahl ≥ 2 ist und wobei zwischen unmittelbar benachbarten Kleinbarren und dem Endlosband eine stoffliche Verbindung bestehen bleibt. Auf diese Weise entsteht ein Endlosverbundbarren. Zusätzlich weist das Endlosband eine Breite B größer als die Breite b des herzustellenden Barrens auf, sodass beim Umformen des Endlosbandes zusätzlich zu dem Barren ein überstehender Rand entsteht.

Wenn bei der Umformung ein automatisches Abtrennen des Randes erfolgt, kann nach einer Herstellung einer gewünschte Zahl von Reihen eine Abtrennung vom Endlosband erfolgen und der fertige Barren liegt dann vor.

Wenn das umgeformte Endlosband zur Herstellung des Barrens nach einer vorgegebenen Zahl von Reihen im Bereich der stofflichen Verbindung zwischen einer Reihe und dem Endlosband getrennt wird, kann der gewünschte Barren entstehen.

Vorteilhafterweise kann der beim Umformen entstehende Kleinbarren gleichzeitig beschriftet werden.

### Kurzbeschreibung der Zeichnung

In der Zeichnung sind Ausführungsbeispiele der Erfindung dargestellt. Es zeigt:
- Fig. 1: einen Barren aus Edelmetall in Form einer Tafel mit 4 x 3 Kleinbarren;
- Fig. 2: ein Detail aus Fig. 1;
- Fig. 3: einen Teilschnitt entlang der Linie AA aus Fig. 2;
- Fig. 4A bis 4D: verschiedene Formen einer stofflichen Verbindung zwischen den Kleinbarren einer Tafel;
- Fig. 5A bis: 5D weitere Formen einer stofflichen Verbindung zwischen den Kleinbarren einer Tafel;
- Fig. 6: auf einem Träger angeordnete Kleinbarren ohne stoffliche Verbindungsstelle.
- Fig. 7: ein im Schnitt dargestelltes Endlosband mit Kleinbarren;
- Fig. 8: eine Draufsicht auf das umgeformte Endlosband aus Fig. 7;
- Fig. 9: Kanten eines Werkzeugs für die Herstellung von Kleinbarren.

### Ausführungsbeispiele der Erfindung

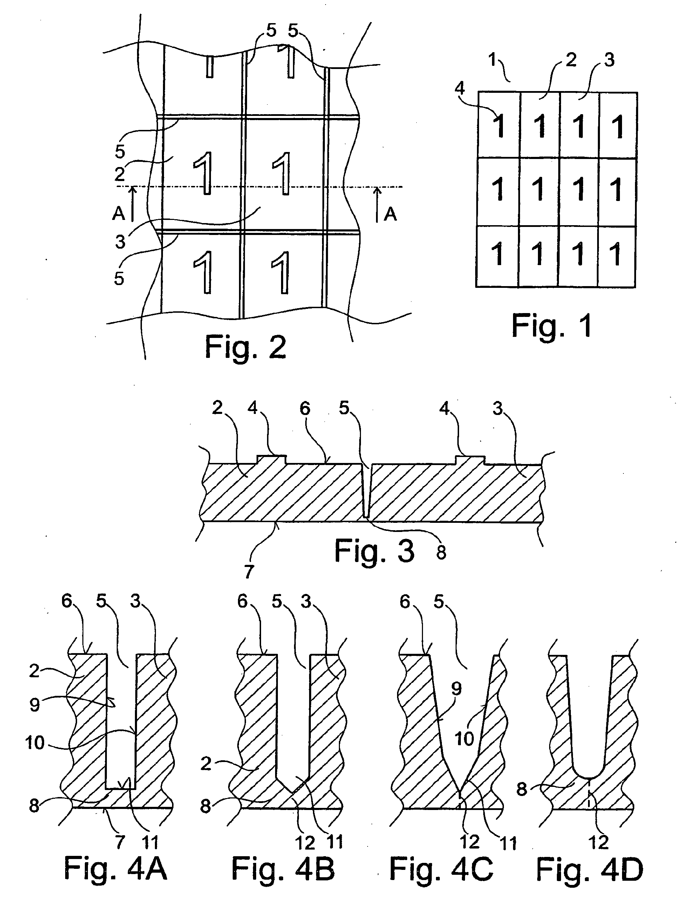

In Fig. 1 ist ein Barren 1 aus Edelmetall in Form einer Tafel mit 4 x 3 Kleinbarren 2,3 gezeigt. Dafür wurde zunächst ein nicht dargestellter Barren in Form einer Tafel mit einer Sollmasse mB hergestellt. Die Bearbeitung des Barrens zur Bereitstellung vieler Kleinbarren 2,3 kann durch Umformen wie beispielsweise Prägen erfolgen, und zwar bereits mit einer Umformung wie einem Prägevorgang, bei dem auch die Angaben 4 über den Kleinbarren 2,3 erzeugt werden, nämlich ein Herstellerlogo, die Masse und die Reinheit, so dass dann die Herstellung einem einzigen Arbeitsgang erfolgen kann. Im Ausführungsbeispiel ist als Angabe 4 die Masse mit 1 angegeben, was 1g bedeutet.

Wie in Fig. 2 im Detail dargestellt, wurden durch den Umformvorgang in den Barren 1 in vorgegebenen Abständen Vertiefungen in Form von Rillen 5 eingebracht, die einen einzelnen Kleinbarren 2 von dem benachbarten Kleinbarren 3 klar erkennbar abgrenzen. Die Lage der Rillen 5 in dem Barren 1 ist so gewählt, dass die durch die Rillen 5 begrenzten Kleinbarren 2,3 die gewünschte Masse aufweisen.

Der Barren kann vor dem Umformvorgang vorzugsweise eine vorgegebene gleichmäßige Dicke aufweisen, damit die Umformung des Barrens mit einem einzigen Prägewerkzeug vorgenommen werden kann und die Masse für die Kleinbarren 2,3 genau genug, also innerhalb der zulässigen Toleranzen, erreicht wird.

Wie ein Barren als Tafel hergestellt werden kann, wird am Beispiel eines Feingoldbarrens mit einer Masse von 100 g erläutert, der in 100 Kleinbarren zu je 1 g unterteilt wird. Dazu wird ein Endlosblechband aus 99,99% Feingold auf eine vorher berechnete Dicke ausgewalzt. Aus diesem Band werden Tafeln von je 100g ausgestanzt.

Diese Tafeln werden in einer für die Prägung von Münzen in ähnlicher Weise bereits bekannten Prägemaschine in einem Arbeitsschritt so geprägt, dass Vertiefungen in Form von Rillen 5 zwischen den einzelnen Kleinbarren entstehen, und dass auf jedem einzelnen 1 g Kleinbarren das Herstellerlogo, das Gewicht und die Reinheit eingeprägt werden.

Die als Rillen ausgebildeten Vertiefungen 5, 5' können so dünn ausgeführt werden, dass das verdrängte Material nur einen vergleichsweise kleinen seitlichen Wulst ergibt, welcher durch den Prägevorgang der Flächen aber sofort wieder abgeflacht werden kann, wenn es gewünscht ist. Dünn bedeutet in diesem Zusammenhang, dass die Breite der Vertiefung geringer als deren Tiefe ist und vorzugsweise höchstens 50% der Tiefe beträgt.

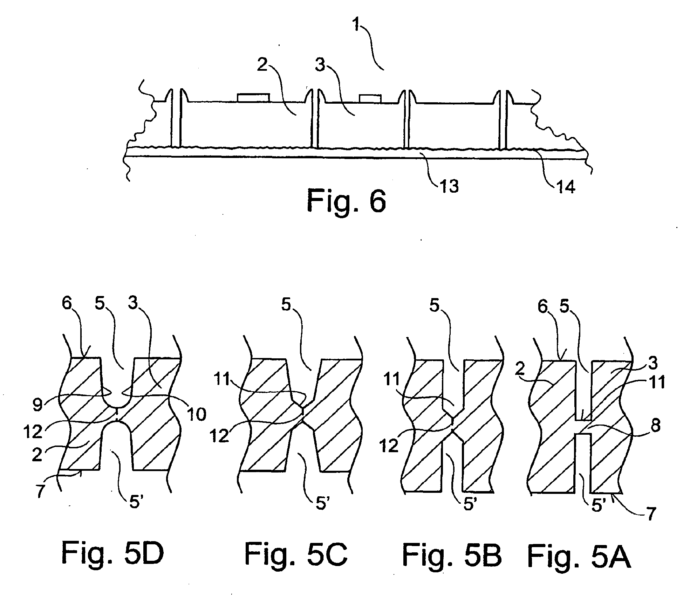

Fig. 3 zeigt einen Teilschnitt entlang der Linie AA aus Fig. 2 und es wird erkennbar, dass die Vertiefungen 5 nicht durch die ganze Tafel hindurch gehen, sondern dass eine von einer Oberseite 6 in Richtung zu einer Unterseite 7 hin ausgehende Rille 5 ausgebildet ist, wobei die stoffliche Verbindung 8 in Form eines Verbindungsstegs, siehe später Fig. 4A-4D, oder einer Brücke, siehe später Fig. 5A-5D besteht.

Die stoffliche Verbindung 8 in der Vertiefung 5 kann dabei in unterschiedlicher Weise ausgebildet sein, wie in den Fig. 4A-4D und 5A-5D beispielhaft gezeigt ist.

In Fig. 4A sind Seitenwände 9,10 der nur von der Oberseite der Tafel her eingebrachten Vertiefung 5 im wesentlichen parallel zueinander und quer zur Oberseite 6 und der Unterseite 7 der Tafel, wobei ein Boden 11 der Vertiefung 5 eben ist und parallel zur Unterseite 7 verläuft. Der Boden 11 ist dabei Teil des Verbindungsstegs der stofflichen Verbindung 8 der benachbarten Kleinbarren 2,3 untereinander.

In Fig. 4B ist im Gegensatz zu Fig. 4A der Boden 11 der Vertiefung zur Unterseite hin zulaufend ausgebildet, sodass in der größten Tiefe des Bodens 11 wegen des geringsten Querschnitts des Verbindungsstegs eine Sollbruchstelle 12 ausgebildet ist.

Mit einer Sollbruchstelle 12 lässt sich die stoffliche Verbindung 8 in der Vertiefung 5 im Bedarfsfall ohne Verwendung von Werkzeug lösen und die Kleinbarren können von einander abgetrennt werden. Dabei wird aufgrund der bekannten Lage der Sollbruchstelle eine planmäßige Verteilung der Massen sichergestellt.

In Fig. 4C sind die Seitenwände der nur von der Oberseite 6 der Tafel her eingebrachten Vertiefung 5 zum Boden hin schräg zulaufend ausgebildet, wobei der Boden 11 der Vertiefung 5 wie in Fig. 4B spitz zulaufend ausgebildet ist. Dadurch ergibt sich eine Sollbruchstelle 12. Im Querschnitt ergibt sich ein Verlauf mit zwei unterschiedlichen Öffnungswinkeln der Vertiefung, wobei der Öffnungswinkel des Bodens 11 größer ist als der Öffnungswinkel der Seitenwände 9,10.

In Fig. 4D sind die Seitenwände wie in Fig. 4C ausgebildet, wohingegen der Boden 11 gerundet ausgebildet ist, etwa als Rinne. Die Sollbruchstelle 12 liegt auch hier im Bereich des kleinsten Querschnitts des Verbindungsstegs 8.

In den Fig. 5A-5D sind weitere Formen einer stofflichen Verbindung 8 zum Teil mit Sollbruchstelle 12 zwischen den Kleinbarren 2,3 einer Tafel dargestellt, die sich von denen der Fig. 4A-4D dadurch unterscheiden, dass die stoffliche Verbindung in einer sowohl von der Oberseite 6 als auch von der Unterseite 7 eingebrachten Vertiefung 5,5' angeordnet ist. In diesem Fall wird das Material sowohl an die Oberseite 6 als auch an die Unterseite 7 verdrängt und kann, falls gewünscht, im Prägevorgang abgeflacht werden.

In Fig. 6 ist die Tafel 1 mit ihrer Unterseite 7 auf einem Träger 13 angeordnet und mit diesem beispielsweise durch Kleben verbunden, sodass selbst nach einem vollständigen Trennen der Kleinbarren, die dadurch also ohne stoffliche Verbindungsstelle untereinander sind, ein Zusammenhalt gegeben ist. Die stoffliche Trennung kann durch Einbringen einer Vertiefung 5 in Form einer Rille erfolgen, wobei die Vertiefung von der Oberseite 6, also der dem Träger 13 gegenüberliegenden Seite der Tafel ausgeht.

Als Träger 13 kommt beispielsweise eine Trägerplatte in Frage, die mit einer Gummierung 14 ähnlich wie bei Aufklebern beschichtet ist, von der sich die einzelnen Kleinbarren 2,3 leicht ablösen lassen, ohne dass Klebespuren auf den Kleinbarren 2,3 verbleiben. Die Trägerplatte kann entweder aus dickerem Papier, einem Karton oder Kunststoff bestehen.

Die Kleinbarren 2,3 lassen sich dann einzeln oder zu mehreren von dem Träger lösen, die nicht gelösten übrigen Kleinbarren bleiben auf dem Träger 13 und sind wieder gemeinsam handhabbar.

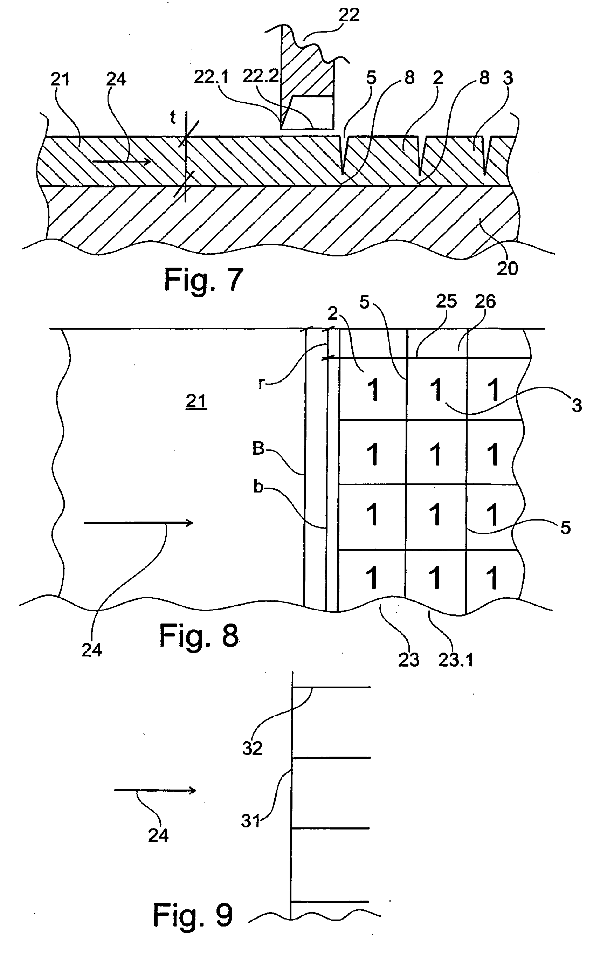

In einer weiteren, in den Fig. 7-9 dargestellten Ausführungsform wird der Barren 1 so hergestellt, dass ausgehend von einem in Fig. 7 im Schnitt dargestellten, auf eine vorberechnete Dicke t ausgewalztes Endlosband 21 aus 99,99% Feingold eine schrittweise Umformung des Endlosbandes 21 unter Ausbildung der Kleinbarren erfolgt.

Dabei wird das auf einem Tisch 20 aufliegende Endlosblechband 21 an eine Umformstation 22 herangeführt und in einem Umformschritt werden Kleinbarren 2 erzeugt, die mit einem im vorherigen Umformschritt erzeugten Kleinbarren 3 und zusätzlich auch noch mit dem noch nicht umgeformten Endlosband 21 jeweils am Boden einer als Rille ausgebildete Vertiefung 5 eine stoffliche Verbindung 8 aufweisen. Die als Rille ausgebildete Vertiefung 5 wird mit einer scharfen Kante 22.1 erzeugt, eine weitere scharfe Kante 22.2 ist für nicht dargestellte Rillen in Zufuhrrichtung 24 gezeigt.

Wie aus der Draufsicht aus Fig. 8 ersichtlich ist, sind die Kleinbarren 2, 3 in jeweils einer Reihe 23, 23.1. quer zu der Zufuhrrichtung 24 des Endlosbandes 21 angeordnet und durch die als Rille ausgebildete Vertiefung 5 voneinander abgesetzt.

Ist die Umformung einer Reihe 23 abgeschlossen, wird das Endlosblechband 21 gegenüber der nicht dargestellten Umformeinrichtung einen Schritt weiter vorgeschoben und es findet eine erneute Umformung statt, um die nächste Reihe von Kleinbarren 2, 3 zu erzeugen. Der Barren selbst kann dann durch geeignetes Abtrennen auf die gewünschte Anzahl von Reihen 23 von Kleinbarren 2, 3 gebracht werden.

Wenn das Endlosblechband 21 eine Breite B größer als die Breite b des herzustellenden Barren aufweist, wird jeder Kleinbarren 2, 3 bei seiner Herstellung denselben Umformungen unterworfen. Allerdings entsteht durch das seitliche Übermaß des Endlosblechbandes 21 gegenüber dem Barren 1 dann ein durch eine als Rille ausgebildete Vertiefung 25 abgesetzter aber gleichwohl noch stofflich verbundener seitlicher Rand 26 der Breite r, der sich in Zufuhrrichtung 25 erstreckt. Der Rand 26 kann bereits beim Umformen abgetrennt werden oder er wird erst nach dem Umformen abgetrennt.

Auch bei dieser Umformung kann die Erzeugung von Vertiefungen 5 sowohl von der Oberseite her als auch von der Unterseite her des Endlosbandes 21 erfolgen. Die zusätzliche Umformung von der Unterseite her ist grundsätzlich dann vorteilhaft, wenn die Dicke des Barrens so groß ist, dass die Umformung von nur einer Seite her nicht ausreicht.

Die durch die Einbringung von Vertiefungen 5 in den Barren erzeugten Sollbruchstellen im Bereich der stofflichen Verbindung 8 können einen Öffnungswinkel von etwa 10° bis 60° aufweisen und die stoffliche Verbindung 8 kann eine Dicke von 0,05mm bis 0,4mm aufweisen, wobei auch andere Dicken noch ein Abtrennen von Hand ermöglichen.

In Fig. 9 sind Kanten 31, 32 eines Werkzeugs für die Herstellung von Kleinbarren dargestellt. Eine Kante 31 verläuft quer zur Zuführrichtung 24 und erzeugt die als Rille ausgebildete Vertiefung 5, eine weitere Kante 32 in Zuführrichtung 24 erzeugt beispielsweise die als Rille ausgebildete Vertiefung 25 am Rand 26 eines Kleinbarrens 2, 3 aus Fig. 8. Die Kanten 231, 32 werden in das Endlosband gedrückt und verdrängen das Material, sodass eine Vertiefung entsteht, wobei gleichzeitig eine stoffliche Verbindung bestehen bleibt, was hier nicht dargestellt ist.

Auch bei der Ausführungsform nach den Fig. 7-9 ist es möglich, anstelle einer stofflichen Verbindung ein Träger vorzusehen, der mit dem Endlosband verbunden ist.

Die Barren werden beispielsweise in den nachfolgend angegebenen Größen hergestellt. Für Gold 100x1g: 74mm x 105mm x 0,667mm oder 85mm x 150mm x 0,406mm; für Silber: 100x1g: 74mm x 105mm x 1,226mm; für Platin: 100x1g: 74mm x 105mm x 0,602mm und für Palladium: 100x1g : 74mm x 105mm x 1,073mm.

Es lassen sich hier also zwei Herstellungsvarianten unterscheiden, wobei andere Herstellungsverfahren, etwa durch Gießen, nicht ausgeschlossen werden. In der ersten Variante wird ein Edelmetallblech auf die fertigen Endmaße geschnitten. Das zugeschnittene Blech kommt dann in eine normale Prägemaschine, wie sie auch für Münzen oder normale Edelmetallbarren eingesetzt wird und wird dort mit entsprechend ausgeformten Prägestempeln unter hohem Druck in die Endform geprägt.

In der zweiten Variante wird ein Edelmetall-Endlosband in entsprechender Dicke gewalzt und in einer Stanzmaschine jeweils eine komplette Reihe, etwa 10 Kleinbarren zu 1g gleichzeitig gekerbt und beschriftet. Danach wird das Band weitergeschoben, so dass ein Endlosverbund entsteht, der dann jeweils nach 10 Reihen gekappt werden kann, um einen Barrenverbund in einer Stückelung von 10x10x1g zu erhalten.

Mit beiden Varianten können auch hochglänzende Oberfläche hergestellt werden.

## Patentansprüche

1. Barren aus Edelmetall oder einer Edelmetall aufweisenden Legierung, mit einer Masse mB, **dadurch gekennzeich-net, dass** der Barren (1) in n x m Kleinbarren (2,3) mit jeweils einer vorgegebenen Masse mk unterteilt ist, wobei n, m jeweils eine natürliche Zahl ≥ 2 ist und dass zwischen den unmittelbar benachbarten Kleinbarren (2,3) eine stoffliche Verbindung (8) besteht.

2. Barren nach Anspruch 1, **dadurch gekennzeichnet, dass** die stoffliche Verbindung (8) eine Sollbruchstelle (12) aufweist.

3. Barren nach einem der Ansprüche 1 bis 2, **dadurch gekennzeichnet, dass** auf einer Unterseite (7) des Barren ein Träger (13) angebracht ist.

4. Barren nach einem der Ansprüche 1 bis 3, **dadurch gekennzeichnet, dass** die stoffliche Verbindung (8) Teil einer in den Barren (1) eingebrachten Vertiefung (5,5') ist.

5. Barren nach Anspruch 4, **dadurch gekennzeichnet, dass** eine Vertiefung (5) auf einer Oberseite (6) und eine Vertiefung (5') gegenüberliegend auf einer Unterseite (7) ausgebildet ist und dass die stoffliche Verbindung (8) zu der Oberseite (6) und zu der Unterseite (7) des Barrens beabstandet ist.

6. Barren nach Anspruch 2 und 4, **dadurch gekennzeichnet, dass** die stoffliche Verbindung (8) Teil einer in den Barren (1) eingebrachten, zulaufend ausgebildeten Vertiefung (5,5') ist und dass die stoffliche Verbindung (8) in der größten Tiefe der Vertiefung eine Sollbruchstelle (12) aufweist und eine Dicke aufweist, die ein Abtrennen von Hand ermöglicht.

7. Barren aus Edelmetall oder einer Edelmetall aufweisenden Legierung, mit einer Masse mB, **dadurch gekennzeich-net, dass** der Barren in n x m Kleinbarren (2,3) mit jeweils einer vorgegebenen Masse mk unterteilt ist, wobei n, m jeweils eine natürliche Zahl ≥ 2 ist und dass auf einer Unterseite (7) des Barren ein Träger (13) angebracht ist und dass die Kleinbarren (2,3) umfangseitig vollständig zu den benachbarten Kleinbarren frei sind und ausschließlich an dem Träger (13) befestigt sind.

8. Barren nach Anspruch 7, **dadurch gekennzeichnet, dass** die Kleinbarren (2,3) mittels einer den Barren bis zum Träger (13) durchdringenden Vertiefung (5) von einander abgetrennt sind.

9. Barren nach einem der Ansprüche 4 bis 6 oder 8, **dadurch gekennzeichnet, dass** die Vertiefung eingeprägt ist.

10. Verfahren zur Herstellung eines Barren mit einer Masse mB aus Edelmetall oder einer Edelmetall aufweisenden Legierung, **dadurch gekennzeichnet, dass** der Barren (1) in einem Herstellungsschritt in n x m Kleinbarren (2,3) mit jeweils einer vorgegebenen Masse mk unterteilt wird, wobei n, m jeweils eine natürliche Zahl ≥ 2 ist, wobei zwischen den unmittelbar benachbarten Kleinbarren (2,3) eine stoffliche Verbindung (8) bestehen bleibt.

11. Verfahren zur Herstellung eines Barren mit einer Masse mB aus Edelmetall oder einer Edelmetall aufweisenden Legierung, **dadurch gekennzeichnet, dass** der Barren in einem Herstellungsschritt mit einem Träger (13) verbunden wird und in einem weiteren Herstellungsschritt in n x m Kleinbarren (2,3) mit jeweils einer vorgegebenen Masse mk unterteilt wird, wobei n, m jeweils eine natürliche Zahl ≥ 2 ist, wobei die Kleinbarren (2,3) umfangseitig vollständig zu den benachbarten Kleinbarren (2,3) frei werden und ausschließlich an dem Träger (13) befestigt sind.

12. Verfahren zur Herstellung eines Barren mit einer Masse mB aus Edelmetall oder einer Edelmetall aufweisenden Legierung, bei dem ein Edelmetall-Endlosband in einer entsprechenden Dicke gewalzt wird und einer Umformeinrichtung schrittweise zugeführt und nach einer Umformung das Band weitergeschoben wird, **dadurch gekennzeichnet, dass** bei der Umformung das Endlosband in eine Reihe (23; 23.1) aus n x 1 Kleinbarren (2; 3) mit jeweils einer vorgegebenen Masse mk unterteilt wird, wobei n eine natürliche Zahl ≥ 2 ist, wobei zwischen unmittelbar benachbarten Kleinbarren (2, 3) und dem Endlosband (21) eine stoffliche Verbindung (8) bestehen bleibt, wobei das Endlosband (21) eine Breite B größer als die Breite b des herzustellenden Barrens (1) aufweist, sodass beim Umformen zusätzlich zu dem Barren (1) ein überstehender Rand (26) entsteht.

13. Verfahren nach Anspruch 12, **dadurch gekennzeichnet, dass** das umgeformte Endlosband zur Herstellung des Barrens nach einer vorgegebenen Zahl von Reihen (23, 23.1) im Bereich der stofflichen Verbindung (8) zwischen einer Reihe (23) und dem Endlosband (21) getrennt wird.

14. Verfahren nach einem der Ansprüche 10 bis 13, **dadurch gekennzeichnet, dass** der Kleinbarren bei seiner Herstellung gleichzeitig beschriftet wird.

## Claims

1. A bar of noble metal or an alloy containing noble metal having a mass mB, **characterized in that** said bar (1) is subdivided into n x m miniature bars (2, 3) each having a specified mass mk, wherein n and m each denote an integer ≥ 2, and that there is an interconnection of solid material (8) between the directly adjacent miniature bars (2, 3).

2. The bar as defined in claim 1, **characterized in that** said interconnection of solid material (8) has a predetermined break point (12).

3. The bar as defined in any one of claims 1 to 2, **characterized in that** a backing (13) is attached to the underside (7) of said bar.

4. The bar as defined in any one of claims 1 to 3, **characterized in that** said interconnection of solid material (8) is part of a depression (5,5') formed in said bar (1).

5. The bar as defined in claim 4, **characterized in that** there is a depression (5) on the top surface (6) and a depression (5') opposite thereto on the underside (7), and that said interconnection of solid material (8) is set at a distance from said top surface (6) and from the underside (7) of said bar.

6. The bar as defined in claim 2 and claim 4, **characterized in that** said interconnection of solid material (8) is part of a tapered depression (5, 5') formed in said bar, and that said interconnection of solid material (8) has a predetermined break point (12) in the deepest region of said depression and has a thickness enabling manual break-off.

7. A bar of noble metal or an alloy containing noble metal having a mass mB, **characterized in that** said bar is subdivided into n x m miniature bars (2, 3) each having a specified mass mk, where n, m each denote an integer ≥ 2 and that a backing (13) is attached to the underside (7) of said bar and that said miniature bars (2, 3) are peripherally spaced, all round, from the adjacent miniature bars and are attached exclusively to said backing (13).

8. The bar as defined in claim 7, **characterized in that** said miniature bars (2, 3) are separated from each other by means of a depression (5) penetrating the bar down to said backing (13).

9. The bar as defined in any one of claims 4 to 6 or claim 8 **characterized in that** the depression is impressed.

10. A method for the production of a bar having a mass mB consisting of noble metal or an alloy containing noble metal, **characterized in that,** in one manufacturing step, the bar (1) is divided up into n x m miniature bars (2, 3) each having a specified mass mk, wherein n and m each denote an integer ≥ 2, while an interconnection of solid material (8) remains between directly adjacent miniature bars (2, 3).

11. A method for the production of a bar having a mass mB consisting of noble metal or an alloy containing noble metal, **characterized in that** the bar is attached, in one manufacturing step, to a backing (13) and, in another manufacturing step, is divided up into n x m miniature bars (2, 3) each having a specified mass mk, wherein n and m each denote an integer ≥ 2, which miniature bars (2, 3) are peripherally spaced, all round, from the adjacent miniature bars (2, 3) and are attached exclusively to said backing (13).

12. A method for the production of a bar having a mass mB of noble metal or an alloy containing noble metal in which a continuous band of noble metal is rolled to a desired thickness and is fed stepwise to a shaping device and moved away therefrom following shaping, **characterized in that** during said shaping the continuous band is divided up into a row (23, 23.1) of n x 1 miniature bars (2, 3) each having a specified mass mk, where n denotes an integer ≥ 2, an interconnection of solid material (8) being left between directly adjacent miniature bars (2, 3) and said continuous band (21), wherein said continuous band (21) has a width B which is greater than the width b of the bar (1) to be fabricated such that shaping produces, in addition to the bar (1), a protruding edge (26).

13. The method as defined in claim 12, **characterized in that**, during the production of said bar, the shaped continuous band is severed in the region of the interconnection of solid material (8) between a row (23) and said continuous band (21) following the creation of a specified number of rows (23, 23.1).

14. The method as defined in any one of claims 9 to 11 **characterized in that** said miniature bar is inscribed during production thereof.

## Revendications

1. Lingot en métal noble ou en alliage contenant du métal noble, ayant un poids mB, **caractérisée en ce que** le lingot (1) est divisée en n x m petits lingots (2, 3) ayant chacun une masse prédéfinie mk, n, m étant respectivement un nombre naturel ≥ 2, et que, entre les petits lingots (2, 3) directement voisins, une liaison matérielle (8) existe.

2. Lingot selon la revendication 1, **caractérisée en ce que** la liaison matérielle (8) présente un point de rupture théorique (12).

3. Lingot selon une des revendications 1 à 2, **caractérisée en ce qu'un** support (13) est installé sur une face inférieure (7) du lingot.

4. Lingot selon une des revendications 1 à 3, **caractérisée en ce que** la liaison matérielle (8) fait partie d'une cavité (5, 5') pratiquée dans le lingot (1).

5. Lingot selon la revendication 4, **caractérisée en ce qu'une** cavité (5) est pratiquée sur une face supérieure (6) et une cavité (5') à l'opposé sur une face inférieure (7) et que la liaison matérielle (8) est espacée de la face supérieure (6) et de la face inférieure (7) du lingot.

6. Lingot selon les revendications 2 et 4, **caractérisée en ce que** la liaison matérielle (8) fait partie d'une cavité (5, 5') effilée pratiquée dans le lingot (1) et que la liaison matérielle (8) présente au plus profond de la cavité un point de rupture théorique (12) et une épaisseur qui permet un détachement à la main.

7. Lingot en métal noble ou en alliage contenant du métal noble, ayant un poids mB, **caractérisée en ce que** le lingot est divisée en n x m petits lingots (2, 3) ayant chacun une masse prédéfinie mk, n, m étant respectivement un nombre naturel ≥ 2, et que, sur une face inférieure (7) du lingot, un support (13) est installé et que les petits lingots (2, 3) sont, au niveau de leur circonférence, entièrement libres par rapport aux petits lingots voisines et exclusivement fixées au support (13).

8. Lingot selon la revendication 7, **caractérisée en ce que** les petits lingots (2, 3) sont séparées entre elles par une cavité (5) traversant le lingot jusqu'au support (13).

9. Lingot selon une des revendication 4 à 6 ou 8, **caractérisée en ce que** la cavité est réalisée par monnayage.

10. Procédé de fabrication d'un lingot en métal noble ou en alliage contenant du métal noble, **caractérisé en ce que** le lingot (1) est divisé lors d'un stade de fabrication en n x m petits lingots (2, 3) ayant chacun une masse prédéfinie mk, n, m étant respectivement un nombre naturel ≥ 2, une liaison matérielle (8) étant conservée entre les petits lingots (2, 3) directement voisins.

11. Procédé de fabrication d'un lingot en métal noble ou en alliage contenant du métal noble, ayant un poids mB, **caractérisé en ce que** le lingot est reliée à un support (13) lors d'un stade de fabrication et divisée en n x m petits lingot (2, 3) ayant chacun une masse prédéfinie mk, n, m étant respectivement un nombre naturel ≥ 2, les petits lingots (2, 3) devenant totalement libres au niveau de leur circonférence par rapport aux petits lingots (2, 3) voisins et étant fixés exclusivement au support (13).

12. Procédé de fabrication d'un lingot ayant un poids mB en métal noble ou en alliage contenant du métal noble, dans lequel une bande continue de métal noble est laminée à une épaisseur appropriée et acheminée graduellement à un dispositif de déformation et la bande est poussée plus loin après déformation, **caractérisé en ce que,** lors de sa déformation, la bande continue est divisée en une série de n x 1 petits lingots (2, 3) ayant chacun une masse prédéfinie mk, n étant un nombre naturel ≥ 2, une liaison matérielle (8) étant conservée entre les petits lingots (2, 3) directement voisins et la bande continue (21), la bande continue (21) ayant une largeur B supérieure à la largeur b du lingot à fabriquer (1), de telle sorte que, lors de la déformation, un bord proéminent (26) est créé en plus de la barre (1).

13. Procédé selon la revendication 12, **caractérisé en ce que** la bande sans fin déformée pour la fabrication du lingot est coupé après un nombre prédéfini de séries (23, 23.1) au niveau de la liaison matérielle (8) entre une série (23) et la bande sans fin (21).

14. Procédé selon une des revendications 10 à 13, **caractérisé en ce que** le petit lingot reçoit une inscription au moment même de sa fabrication.
